# EUROPEAN PATENT SPECIFICATION

(11) **EP 4 164 545 B1**
(45) Date of publication and mention of the grant of the patent: **01.04.2026**
(21) Application number: 21733908.4
(22) Date of filing: 11.06.2021
(51) Int. Cl.: A61F 2/00

(54) **FLOW CONTROL DEVICE**
DURCHFLUSSREGELVORRICHTUNG
DISPOSITIF DE RÉGULATION DE DÉBIT

(30) Priority: 12.06.2020 GB 202008992
(43) Date of publication of application: 19.04.2023
(73) Proprietor: Nowwell AS, 6823 Sandane (NO)
(72) Inventor: KRISTENSEN, Kim, 5610 Øystese (NO); GRANAT, Leif, 17975 Skå (SE)
(74) Representative: Dehns
(86) International application number: PCT/EP2021/065790
(87) International publication number: WO 2021/250243

(56) References cited:
- WO-A1-2006/115225
- US-A- 3 812 841
- US-A- 5 711 314

## Description

One of the causes of urinary incontinence (UI) - the involuntary leakage of urine - is the loss or weakening of the urethral sphincter which affects approximately 5% of people around the globe. In men, UI is particularly common in people with prostate cancer who have undergone a prostatectomy, affecting approximately 10% of patients undergoing prostatectomy annually in the USA. UI is also a common problem for patients with an enlarged prostate, affecting 30-40% of patients undergoing transurethral resection of the prostate (TURP) annually in the USA. In women, pregnancy and childbirth may lead to weak pelvic floor muscles or damage the surrounding nerves which can make the urethral sphincter incompetent. UI severely affects the quality of life in patients of all ages.

Various methods exist for treating UI, each with its own inherent problems. A common, cheap solution is the use of diapers, which simply absorb any leaked urine, before being disposed of by a user. Diapers do not offer a long-term solution, as they need to be replaced regularly. Additionally, the use of diapers generates excessive waste due to each diaper requiring disposal after one use.

Catheters offer a short-term solution. A catheter is a soft hollow tube which may be passed into the bladder in order to drain urine, among other uses. Catheters offer only a short-term solution, as with long-term use various issues may arise, including urinary tract infections (UTls) and flow disruption due to mineral deposition within the catheter. A catheter also simply directs the urine flow to a receptacle (usually worn) outside the patient's body without providing any control over the flow. Surgically implanted devices are also known, for example that may be inserted into the body via surgery. One such procedure provides a user-controllable urethral constriction cuff in order to control the flow of urine out of the bladder. This provides a solution for some patients, but there are inherent risks with surgery, as well as costs.

US 5 711 314 A relates to a urinary incontinence valve for positioning in a patient's urethra and bladder, and controlling the flow of urine therethrough. The valve includes an annular valve seat surface and a valve member cooperating with the valve seat surface for normally blocking urine flow. A compression spring normally urges valve member into sealing contact relative to the annular valve seat surface with a predetermined force. An actuator rod, supporting a first permanent magnet, is affixed to said valve and projects into the urinary bladder of the patient. A first anchoring mechanism comprises a deflectable spring mechanism which extends into the bladder and contacts an inner bladder surface adjacent an opening of the bladder into the urethra. A second anchoring mechanism comprising a retaining spring is positionable proximate the urethra meatus. The first and second anchoring mechanisms prevent migration of the urinary incontinence valve after it is properly positioned in the patient.

The invention is as set forth in the appended claims.

According to a first aspect, the invention provides a device for controlling urinary flow, comprising:
a fluid inlet;
a fluid outlet;
a valve movable between an open position in which fluid can flow from the fluid inlet to the fluid outlet and a closed position in which fluid flow is blocked between the fluid inlet and the fluid outlet; and
an actuator operable to move the valve between the open position and the closed position;
wherein the actuator is positioned on a first side of the fluid inlet; and
wherein the fluid outlet is positioned on a second, opposite side of the fluid inlet.

In use, the device allows the flow of urine from the bladder through the urethra to be controlled by operation of the valve. The fluid inlet is arranged inside the bladder so that urine within the bladder can enter the device through the fluid inlet. When the valve is in the closed position, further onward flow of urine is blocked such that urine remains held within the bladder. When the valve is in the open position, a flow path is formed between the inlet and the outlet such that urine in the bladder can flow into the device through the fluid inlet, past the valve and out of the device through the fluid outlet so as to empty urine from the bladder. The relative positioning of the actuator (with the actuator and the fluid outlet positioned on opposite sides of the fluid inlet), the fluid inlet and the fluid outlet ensures that the actuator does not obstruct the flow path between the fluid inlet and the fluid outlet. The cross-sectional area of the conduit between fluid inlet and outlet can thus be maximised. This allows a high flow rate (i.e. a relatively normal urination flow rate) to be achieved compared with existing devices. The improved flow rate gives an improved quality of life for the user as the urination process is much closer to normal. Operation of the actuator to move the valve to the open position equates to relaxing of the urethral sphincter muscle in a healthy patient. Urination can then proceed at a substantially normal rate until the bladder is empty. The valve can subsequently be closed again to retain urine in the bladder once more.

It will be appreciated that the device is equally applicable to both male and female anatomy. The same device may be used for both males and females. Alternatively, male-specific and female-specific versions may be made with minor adjustments to account for differences in male and female anatomy, e.g. differences of size or length of the outlet tube (discussed below), but with the same function as described in the rest of this document.

The device may comprise an outlet tube extending between the fluid inlet and the fluid outlet. In use the outlet tube may extend within the urethra, positioning the fluid outlet within the urethra for onward travel of urine during urination and also allowing the device to be anchored in position reliably.

The fluid outlet may be considered to be at a proximal end of the device and the actuator may be considered to be towards a distal end of the device, with the fluid inlet positioned between the two. The terms proximal and distal are used relative to the external urethral opening through which the device may be inserted (i.e. the opening by which urine leaves the body). The proximal end is closer to the external urethral opening while the distal end is further away from the external urethral opening.

The outlet tube forms the flow path by which urine leaves the bladder through the device. The outlet tube is therefore preferably of as large a diameter as is practical so as to maximise the flow rate of urine during use. A larger diameter outlet tube provides less restriction to flow rate. The length of the outlet tube is preferably long enough to allow solid anchoring of the device (i.e. long enough to accommodate an anchoring structure) while being no longer than is necessary to achieve this goal (a shorter device is less invasive and is easier to insert). In some embodiments the outlet tube may have a length of at least 1 cm, optionally at least 2 cm. In some embodiments the outlet tube may have a length of no more than 5 cm, optionally no more than 4 cm, optionally no more than 3 cm. In women, the length of the urethra is shorter (about 2 - 2.5 cm), so the outlet tube may be shorter in a women-specific version. In such embodiments the outlet tube may have a length of no more than 2.5 cm, optionally no more than 2 cm. The diameter of the outlet tube (and indeed the whole device) may in some embodiments be no more than 9 mm, optionally no more than 8 mm. In some embodiments it is desirable that the device as a whole should not extend more than 3 cm into the bladder so as to avoid risk of damage to the bladder wall when the bladder is empty. Accordingly, in some embodiments the overall device length may be no more than 8 cm, optionally no more than 7 cm, optionally no more than 6 cm, optionally no more than 5.5 cm, optionally no more than 5 cm.

The valve may be arranged such that in the closed position it closes the fluid inlet or the fluid outlet directly. For example a valve head may be arranged so that in the closed position it is positioned to completely cover and block the fluid inlet so that no fluid can enter the device. Equally the valve head may be arranged so that in the closed position it is positioned to completely cover and block the fluid outlet so that no fluid can exit the device. Alternatively, a valve seat may be located between the fluid inlet and the fluid outlet and arranged such that the valve engages with the valve seat in the closed position. With such arrangements, the valve seat may be specifically shaped and/or adapted for sealing engagement with the head of the valve so that a good and strong seal can be obtained between the two parts. This allows the inlet and outlet to be independently designed for optimal fluid inlet and outlet characteristics while allowing the valve seat to be designed for optimal sealing characteristics. The valve seat may be in the form of a constriction in the flow passage between the inlet and the outlet against which the valve can be pressed so as to close the flow passage and prevent fluid flow therethrough. Ideally such constriction will be only a small reduction in cross-sectional area so that it does not impede flow any more than necessary. However, the provision of a dedicated valve seat does allow a better seal by allowing a greater force to be applied between the valve and the valve seat, thereby reducing or eliminating leakage past the valve. In the present invention, the valve seat is located close to the fluid inlet so that the valve can be moved substantially (or completely) out of the flow path when the valve is in the open position.

In some embodiments the device is elongate, e.g. substantially in the form of a tube, and comprises a first section comprising the actuator and a second section comprising the fluid outlet and wherein the fluid inlet is formed in a side wall of the device at a position between the first section and the second section. An elongate device is well adapted for insertion of the device through the urethra, thereby allowing a non-surgical installation procedure which is far less invasive to the patient and which can be carried out faster and without requiring surgical equipment or experience. This also reduces the costs involved in installation. As discussed above, the inlet in the sidewall is located between the actuator and the outlet as it is located between the first section and the second section. Thus fluid can enter the device through the sidewall of the device, unobstructed by the actuator in the first section, and with a clear flow path towards the outlet in the second section, obstructed only be the valve (and then only if it is in the closed position).

The device preferably has a smooth shape to avoid irritation of the tissues during insertion and/or removal and while in place. For example the device may have a rounded cross-section, e.g. a circular or elliptic cross-section. The device may have a rounded distal end for ease of insertion into the urethra.

The device is preferably insertable into a urethra and arranged such that, in use, the first section is located in the urinary bladder and the second section is located in the urethra. By locating the first section in the bladder rather than in the urethra, the actuator is located in a position in which it does not obstruct fluid flow out of the bladder and into the urethra. Instead, the actuator (and first section) can project away from the urethral opening into the central volume of the bladder while the fluid inlet allows fluid to enter the device underneath the actuator, whereby its flow into the urethra can be controlled by movement of the valve. The second section can therefore be dimensioned to allow a high flow rate as it only needs to contain the fluid outlet (and optionally a valve seat as discussed above). The second section can otherwise provide an unobstructed flow path. Additionally, the second section being located in the urethra provides some anchoring and stability to the device. The fluid outlet is thus positioned in the urethra so that when urine exits the device it simply passes down the urethra as in a normal urination process.

The actuator may be exposed to urine within the bladder. This may be acceptable so long as the components of the actuator are individually protected or resistant to corrosion. However, such protection may increase the cost and/or size or bulk of the components. Therefore in some embodiments the device may comprise a seal arranged between the fluid inlet and the actuator to prevent fluid flowing from the fluid inlet to the actuator. The seal thereby protects the actuator mechanism from contact with urine and from any corresponding corrosion. This improves the lifetime of the actuator as well as improving its reliability (less chance of failure). This is particularly beneficial as urine contains salts which may crystalize and affect the functionality of the actuator.

In some embodiments the actuator is located in a sealed compartment, the sealed compartment at least partially formed by the seal. By locating the actuator within a sealed compartment, the actuator can be completely protected from the urine, i.e. the sealed compartment is a fluid tight sealed compartment. The seal provides one part of the compartment, adjacent to the fluid inlet. The rest of the compartment may be formed in whole or in part from the body of the device (in particular the first section of the device as discussed above). Thus the actuator may be enclosed within the sealed compartment within the first section of the device and is thus located within the bladder when the device is installed.

The seal may take any suitable form that prevents passage of fluid to the actuator. For example an O-ring, grease retainer seal, or other frictional seal may be used to seal around and/or against a stem (or other component) of the valve such that the stem (or other component) can move past the seal, thereby moving the valve between the open position and the closed position. However, frictional seals provide resistance to the movement and therefore increase the amount of energy required to move the valve between the open position and the closed position. The energy requirements of the device can be an important consideration, especially where the actuator is electrically powered, e.g. by a battery or other energy storage device. The more energy that is used in each actuation, the fewer times the device can be operated given a fixed store of energy. Therefore the usable lifetime of the device depends upon its energy consumption. Therefore in certain preferred embodiments a low friction seal is provided.

In some embodiments the seal comprises a membrane extending between the valve and an inner wall of the device, the membrane arranged to deform as the valve moves between the open position and the closed position. In such embodiments, part of the seal moves with the movable part of the valve (valve stem or valve head) while other parts of the seal may remain substantially at rest (fixed to the inner wall of the device). The seal may be formed from a stretchable material that is attached to a moving part of the valve that moves between the open position and the closed position (e.g. the valve stem or valve head). However, the energy required to stretch the material can produce the same problems as with frictional seals.

Therefore in some embodiments the membrane is arranged in a folded configuration such that the membrane is partly folded back on itself so as to create an overlap and such that movement of the valve between the open position and the closed position changes the amount of overlap. In such embodiments the membrane can be deformed (change shape) during the movement of the valve between the open position and the closed position without significant friction and without significant stretching. Instead, the folded part of the membrane rolls back and forth as the valve is moved, providing very little resistance to the movement. Accordingly, such seals have a very low impact on the energy consumption (i.e. they add very little to the amount of energy required to move the valve), thereby increasing the number of times the valve can be actuated by a given amount of energy and increasing the usable lifetime of the device.

In some embodiments the membrane is at least partly in the form of a tube with one end folded back inside itself. One end of the tube may be attached to the inside wall of the device. The other end of the tube may be attached to the valve (e.g. the valve head or the valve stem) so as to move therewith. In particular, the outer end of the tube may be connected to the inner wall of the device and the inner end of the tube (i.e. the end that is folded back inside) may be connected to the valve so as to move therewith.

The membrane may be formed from a conical tube (i.e. a tube that tapers from a larger diameter at one end to a smaller diameter at the other end), so that the smaller diameter end can be more easily folded back inside towards the larger diameter end.

It will be appreciated that the membrane may have a single fold or multiple folds (e.g. it may be folded backwards, then forwards again, any number of times).

The device may further comprise a first positioning structure movable between a stored configuration and a deployed configuration and wherein, in use, the first positioning structure prevents the device from exiting the bladder through the urethra. Such a positioning structure is useful to define the positional relationship between the device and the urethra/bladder. It is desirable that the device not be easily movable once it has been inserted and deployed, particularly so that the fluid inlet is well positioned in the bladder and the fluid outlet is well positioned in the urethra. If the device could slip back down the urethra, the fluid inlet could become blocked, hindering correct operation of the device.

The first positioning structure may generally be one employed in urinary catheters to hold such catheters in place in the bladder. Thus, the first positioning structure could be a wing-shaped structure comprising two or four wings similar to those employed in a Malecot catheter (made by MEDpro Medical). While this structure prevents unwanted withdrawal, it can lead to tissue bridge formation across the wings and/or stones can become entrapped which may make it difficult to extract the device. Alternatively, the first positioning structure could be a balloon that is deflated during insertion of the device, but which can be inflated (e.g. normally with a liquid such as sterile water or saline) once inserted to an appropriate position. The inflated balloon in the bladder can then prevent unwanted withdrawal of the device before the balloon is once again deflated. Thereby the device is retained within the bladder at an appropriate position. A balloon can be inconvenient however, in that it hinders fluid flow around the urethral opening in the bladder (i.e. at the bladder neck). Therefore it would be difficult to position the fluid inlet close to the urethral opening. When the fluid inlet is displaced from the urethral opening, there will be a certain amount of fluid that is retained in a lower portion of the bladder and that cannot be fully voided through the device due to the separation of the fluid inlet from the urethral opening. This is a non-ideal situation and, apart from being inefficient, can cause problems as the residual urine impairs rinsing of the bladder and germs can settle on the inner wall of the bladder and cause infections.

In some embodiments the first positioning structure is designed to contact a bladder wall (e.g. including contacting the bladder neck) when in the deployed configuration. By contacting the bladder wall, the first positioning structure provides a good anchor by which to prevent unintended extraction of the device as movement of the device will be resisted by the contact with the bladder wall.

In some embodiments the first positioning structure comprises one or more arms pivotably mounted at one end to the device and arranged to pivot between the stored configuration and the deployed configuration. As the arms pivot, they can be held in the stored configuration during the insertion process, then deployed into a configuration in which the device is retained within the bladder. The arms can also be pivoted back to the stored position to allow for extraction of the device at a later time. When deployed, the arms may be in contact with the bladder wall as discussed above. When retracted, the arms may be flush with the outer wall of the device to facilitate insertion and/or removal. The arms may be formed from a suitable material which is compatible with body tissues. Examples include polyether ether ketone (PEEK) and titanium.

The one or more arms may each comprise a support strut attached at one end to an intermediate point on the arm and attached in slidable manner at a second opposite end to the device. The support struts can provide additional stability and smooth operation during movement between the deployed and stored configurations. In addition, the struts can provide a mechanism by which the one or more arms can be pulled back to the stored configuration for removal of the device. The struts may also add support to the first section of the device (the section that sticks up into the bladder away from the urethral opening).

In some embodiments the first positioning structure comprises a skirt. It will be appreciated that this may be provided with or without the one or more arms described above. The skirt is preferably formed from an impermeable material and is designed to flare outwardly towards the distal end of the device (i.e. widening in the direction away from the urethral opening). The skirt may be arranged when in the deployed configuration to have a bowl shape that conforms to a bladder wall around a urethra.

Thus, as the bladder fills, the skirt also fills and the pressure of urine in the bladder (due to gravity) will help to press the skirt against the inner wall of the bladder. This arrangement is convenient for improving the seal between the device and the urethral opening and thereby reducing leakage which may be caused by fluid bypassing the device (e.g. passing along the outside wall of the device so as to exit the urethra without passing through the device). The skirt may be formed from a flexible, foldable or stretchable material so that it can move between the stored configuration and the deployed configuration. For example, the natural (unstretched) form of the skirt may be the deployed configuration and the skirt may be suitable folded up so as to place it in the stored configuration during insertion and removal.

In the deployed configuration, the skirt is preferably a continuous impermeable layer such that any fluid held within the skirt will be directed towards the device, unable to pass through the skirt.

The first positioning structure is preferably attached to the device at a position between the fluid inlet and the fluid outlet and adjacent to the fluid inlet. The first positioning structure may thus be arranged when in the deployed configuration to position the fluid inlet adjacent to the urethral opening in the bladder. This arrangement ensures that the fluid inlet is aligned close to the urethral opening and thus significantly reduces (or even eliminates) the amount of residual fluid in the bladder.

The fluid inlet of the device is essentially located at the urethral opening such that fluid can exit the bladder through the device to the same extent as it could through the urethra (in the absence of the device). In embodiments in which the skirt is used, fluid held within the skirt is directed to the fluid inlet adjacent to the urethra such that the interior of the skirt can be completely voided through the fluid inlet of the device, again reducing (or eliminating) residual urine.

The device may further comprise a second positioning structure movable between a stored configuration and a deployed configuration and, in use, the second positioning structure may be arranged to prevent movement of the device towards the bladder. The second positioning structure may be designed to contact the urethra wall when in the deployed configuration. The second positioning structure thus serves to restrict or prevent movement of the device in the direction towards the bladder (i.e. further inside the body). The second positioning structure may be provided with or without the first positioning structure. However, where both the first and positioning structures are provided, they act in opposition to each other; the first positioning structure preventing movement out of the body and the second positioning structure preventing movement into the body. Together they hold the device in the correct position within the urethra and bladder and can ensure that the fluid inlet is optimally placed for voiding the bladder. Where a skirt is used as the first positioning device, the second positioning device also helps to hold the skirt against the bladder wall, thereby assisting with sealing the bladder to reduce or prevent bypass leakage.

The second positioning structure may comprise one or more fins or ribs biased to expand away from the device when in the deployed configuration. The fins or ribs may be biased by being formed from an elastic material that can be compressed into the stored configuration for insertion and/or removal and will naturally expand into the deployed configuration when released from the stored configuration. The fins or ribs may be arranged to move between the stored configuration and the deployed configuration via stretching or compressing the device. For example, compressing a rib may shorten its length axially, resulting in a corresponding bulge in a radial direction (thereby contacting and holding against the urethra wall). In other embodiments fins may be arranged to flare outwardly from the device (in certain embodiments from the second section of the device) so as to contact the urethra wall in the deployed position and arranged to be compressible so that they can be retracted against the device in the stored configuration.

Any type of actuator can be used that is capable of moving the valve between the open position and the closed position. For example a magnetic switch may be used to control the operation of the valve. In some embodiments a motor may be used to drive the valve between the two positions. A motor may be used together with a gear box to reduce the motor speed and increase the torque to an optimal level for sealing of the valve and for overcoming any resistive forces such as may be provided by a seal or membrane that prevents fluid contacting the actuator (such sealing being particularly important where the actuator is electric). If the valve is a linear valve (i.e. one that moves in a straight line between the open position and the closed position) then the rotary motion of the motor may be converted to linear motion by a suitable mechanism such as a threaded screw. Thus, in some embodiments the actuator comprises an electric motor arranged to impart a rotational force to a threaded screw arranged to convert the rotational motion of the motor into a linear motion of the valve in order to move it between the open position and the closed position.

In some embodiments the actuator comprises a plunger comprising a threaded bore arranged to engage with the thread of the threaded screw, the plunger further comprising one or more guiding members configured to slidably engage with one or more stationary guide rails so as to prevent rotation of the plunger when the threaded screw rotates therein. This may enable the rotational force provided by the motor to be converted to a linear motion of the plunger. The plunger may be fixed to the valve, and thus the valve may be caused to move linearly with the plunger. However, it has been found that the energy consumption of a motor may in some embodiments be quite high, reducing the operational lifetime of the device from an isolated energy source (e.g. a battery) and thus also reducing the time the device can stay located inside the body.

In some embodiments the actuator comprises a solenoid arranged to impart a linear force on the valve to move it between the open position and the closed position. A simple solenoid is normally held in one position by continuous application of current and is returned to another position by a biasing device such as a spring. The continuous application of current can also consume significant energy. However such arrangements may be possible where the solenoid default (no energy) position is to close the valve such that current is only required to open the valve while voiding the bladder. However, the application of continuous current over the time period of a few seconds still puts a limit on the operational lifetime. Therefore in some embodiments the solenoid may be a latching solenoid, which is able to maintain two or more set positions without a constant application of electrical power. For example a bi-stable solenoid can be at rest in two positions which may correspond to the open position and the closed position of the valve. Energy is required to move the solenoid between the two positions, but not to hold it in either position. In such latching solenoids, the current required to move the solenoid is generally higher than for a simple (non-latching) solenoid, but needs to be applied for a much shorter time. Therefore the energy consumption of a latching solenoid is less, making it advantageous in this device as the valve can undergo a greater number of movements from a given fixed capacity energy source (e.g. a battery or capacitor).

Accordingly, the actuator may comprise an energy storage device (such as a battery and/or capacitor) and an electronic control circuit. The electronic control circuit may be arranged to control the actuator so as to move the valve between the open position and the closed position. The energy storage device may provide power to a motor or solenoid as discussed above. The circuit is preferably a low power circuit so as to minimise energy consumption and prolong the operational lifetime of the device. It will be appreciated that the energy storage device and electronic control circuit may be sealed from contact with urine. In some embodiments the energy storage device and electronic control circuit may be housed in the sealed compartment discussed above. In some other embodiments, the energy storage device is rechargeable (e.g. a capacitor or a re-chargeable battery).

In some embodiments, the electronic control circuit comprises wireless charging circuitry for charging the energy storage device while the device is installed in a user. This may increase the period of time for which the device may be continuously installed in a user, as the energy level (e.g. battery level) in the device may be topped-up by a user without first requiring removal of the device. The wireless charging circuitry may comprise circuitry for receiving a radio frequency signal and may use the received signal power to generate a current for charging the energy storage device. The inclusion of wireless charging circuitry in the electronic control circuit may reduce the maximum energy storage capacity required by the device. This may allow the use of a physically smaller energy storage device, which in turn may lead to a reduction in the overall physical size of the urinary flow control device, and thus an improvement in user comfort levels.

In some embodiments the energy storage device of the actuator comprises a capacitor. The capacitor may be the only power storage of the device (i.e. there is no battery or other energy storage device), or it may be provided in addition to a battery. The capacitor may be wrapped around one or more other components of the actuator. This may help to reduce the overall size of the urinary control device, thereby improving comfort when installed in a user. In particular, this may help reduce the length of the urinary control device along a longitudinal axis thereof by utilising unoccupied space in a radial direction of the device, or by slightly increasing a radius of the device as a trade-off for the decrease in length. It is particularly desirable to reduce the length of the urinary control device along its longitudinal axis, as bladders may collapse (e.g. when empty) onto the device when installed in a user if the device is too long, thereby causing discomfort to a user or even causing damage to the bladder tissue.

The capacitor may comprise a coil capacitor wrapped helically around one or more other components of the actuator. This provides a particularly thin and space efficient design as the two capacitor plates are located adjacent to one another at the same radius from the longitudinal axis of the device. In other embodiments the capacitor may comprise a cylindrical capacitor wrapped around one or more other components of the actuator. The capacitor may be positioned adjacent along the longitudinal axis of the device to one or more other components of the actuator.

The capacitor may supply power to the solenoid or motor (e.g. by discharging), and may draw power from the battery (if present) in order to charge. The capacitor may be housed in the sealed compartment as described above. The capacitor may be fully charged, or partially charged, before the urinary flow control device is inserted into a user.

By including a capacitor in the device alongside a battery, the overall battery life of the battery may be increased. As a result, a physically smaller battery may be used while providing users with sufficient battery life. This may reduce the physical size of devices in accordance with the present invention and thus improve comfort for users thereof.

The electronic control circuit may be arranged to operate the actuator based on various factors. For example it may comprise a timing component to determine one or more of the valve opening or closing operations. Other sensors may be provided that the electronic circuit can use to trigger operation of the actuator. For example sensors may determine that a safety condition has arisen and that the valve should be opened. However, in some preferred embodiments the electronic control circuit is arranged to receive a wireless signal from an external source and operate the valve in response to said signal. By using a wireless signal to trigger the valve, the user can have control over their urination. For example the external source may be a remote handheld transmitter that the user can manually trigger to send as wireless signal that is received by the electronic control circuit and that causes the valve to move to the open position so that urine can flow out of the bladder. In this way the user can trigger urination deliberately e.g. when he/she feels the urge to do so and remains in control of the timing of urination. This results in an improved quality of life for the user.

The handheld transmitter may be a dedicated transmitter device designed specifically for operation with the urinary flow control device. The handheld transmitter may be battery powered. Alternatively, or in addition, a mobile telephone or smart-watch or other wearable or portable handheld device capable of transmitting wireless signals can be used.

The urinary flow control device may be formed at least in part (e.g. in the sealed compartment) from materials that have low damping of the wireless signal so as to maximise the signal strength reaching the electronic circuit and to reduce the power that must be transmitted from the remote source. The signal may be selected so as to be capable of transmission through the body so as to reach the electronic circuit.

Thus in some embodiments the electronic control circuit is arranged to receive a radio frequency (RF) signal from a remote control that is manually operable by a user of the device. Various frequencies of RF signal may be used such as frequency bands around 125 kHz, 13.56 MHz and/or 400 MHz, although the 400 MHz band has been found to be more sensitive to the tissue environment. Various signal transmission protocols may also be used, although in many embodiments a one-way communication protocol will suffice and this will reduce the component cost and energy consumption of the electronic control circuit.

According to a second aspect, the invention provides a device for controlling urinary flow, comprising:
a fluid inlet;
a fluid outlet;
a valve movable between an open position in which fluid can flow from the fluid inlet to the fluid outlet and a closed position in which fluid flow is blocked between the fluid inlet and the fluid outlet; and
an actuator operable to move the valve between the open position and the closed position;
wherein the device further comprises a first positioning structure movable between a stored configuration and a deployed configuration and wherein, in use, the first positioning structure is designed to contact a bladder wall when in the deployed configuration;
wherein the first positioning structure comprises a skirt.

The various preferred and optional features described above may also be applied equally to this aspect of the invention. However, in particular relation to the feature of the first positioning structure: The skirt may be arranged when in the deployed configuration to have a bowl shape that conforms to a bladder wall around a urethra. The first positioning structure may comprise one or more arms pivotably mounted at one end to the device and arranged to pivot between the stored configuration and the deployed configuration. The one or more arms each comprise a support strut attached at one end to an intermediate point on the arm and attached in slidable manner at a second opposite end to the device. The first positioning structure may be attached to the device at a position between the fluid inlet and the fluid outlet and adjacent to the fluid inlet. The first positioning structure may be arranged when in the deployed configuration to position the fluid inlet adjacent to a urethral opening in the bladder. The device may further comprise a second positioning structure movable between a stored configuration and a deployed configuration and wherein, in use, the second positioning structure is designed to contact a urethra wall when in the deployed configuration. The second positioning structure may comprise one or more fins or ribs biased to expand away from the device when in the deployed configuration.

The above description has all been provided in relation to a device for controlling urinary flow. However, the general concept of the device may also be applied more generally to other flow control scenarios. For example, on a larger scale the technology may be used for controlling fluid flow into or out of an oil well (e.g. a subsea oil well). Certain embodiments of the invention can be used in other fields where there is a need to control the flow of liquids, e.g. in tanks for storing a liquid like oil, liquid chemicals or water to close and open certain openings in the tank to control the flow of fluid therethrough.

Therefore, according to a further aspect, the invention provides a device for controlling flow, comprising:
a fluid inlet;
a fluid outlet;
a valve movable between an open position in which fluid can flow from the fluid inlet to the fluid outlet and a closed position in which fluid flow is blocked between the fluid inlet and the fluid outlet; and
an actuator operable to move the valve between the open position and the closed position;
wherein the actuator is positioned on a first side of the fluid inlet; and
wherein the fluid outlet is positioned on a second, opposite side of the fluid inlet.

It will be appreciated that the preferred and optional features described above can also be applied and adapted to this more general aspect of the invention.

Certain embodiments of the invention will now be described, by way of example only, and with reference to the accompanying drawings in which:
Fig. 1a shows a longitudinal cross section of a device for controlling urinary flow in accordance with the present invention;
Fig. 1b shows a longitudinal cross section of fluid flow through a device for controlling urinary flow in accordance with the present invention;
Fig. 2 shows a cross section of a user's body with a device for controlling urinary flow positioned therein in accordance with the present invention;
Figs. 3a and 3b show an example of a solenoid valve actuator in accordance with an embodiment of the present invention;
Fig. 4 shows schematic diagrams of examples of a first positioning structure in accordance with the present invention;
Fig. 5 shows examples of a second positioning structure in accordance with the present invention;
Figs. 6a and 6b shows an example membrane seal in accordance with the present invention;
Fig. 7 is a flowchart illustrating the process by which a user may operate a device for controlling urinary flow in accordance with the present invention;
Fig. 8 is a flowchart illustrating the process by which a device for controlling urinary flow in accordance with the present invention may be inserted into a desired position in a user's body; and
Fig. 9 is a flowchart illustrating the process by which a device for controlling urinary flow in accordance with the present invention may be removed from a user's body.
Figs. 10a and 10b show an example of a screw-based valve actuator in accordance with an embodiment of the present invention.
Fig. 11 shows an example of a screw thread valve actuator comprising a coil capacitor in accordance with an embodiment of the present invention.

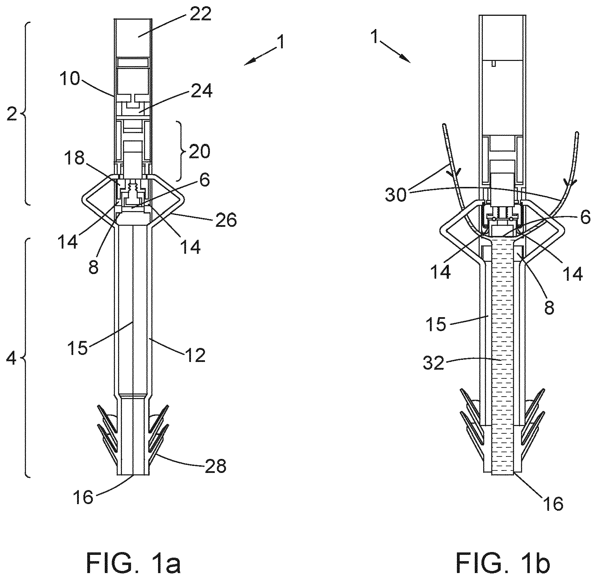

Fig. 1a shows a longitudinal cross section of an embodiment of a urinary flow control device 1 in accordance with the present invention. The device 1 comprises a first section 2 and a second section 4, with a valve 6 and valve seat 8 positioned between the first section 2 and the second section 4. The first section 2 comprises a first casing 10, and the second section 4 comprises a second casing 12. The first casing 10 may comprise any biocompatible, impermeable material, however in this example the first casing 10 comprises polyether ether ketone (PEEK) due to its excellent biocompatibility, mechanical and flexible manufacturing properties. The second casing 12 may also comprise any biocompatible, impermeable material, and in this example comprises the same material (PEEK) as first casing 10 and is formed as an extension of the first casing 10 (i.e. the two casings 10, 12 are integrally formed as a single structure). In other examples, the second casing 12 may be formed as a separate part rather than an extension of the first casing 10.

The second casing 12 is designed to provide support to the first casing 10 that, when in use, is likely to experience at least some level of bending force. Thus the material(s) used for the first casing 10 and the second casing 12 are ideally sufficiently stiff and hard in order to withstand these bending forces.

Adjacent to the valve 6 and valve seat 8 are fluid inlet holes 14 disposed in the side wall of the casing 10 that allow surrounding fluid to flow into the device. Fig. 1a shows the valve 6 in a closed position, making contact with the valve seat 8, wherein fluid is able to enter the inlet holes 14 but is prevented from flowing to the second section 4 by the valve 6 and valve seat 8. In this example the valve seat 8 has at least one orifice disposed therein to allow fluid to flow through when the valve 6 is in an open position. It will be understood by those skilled in the art that the valve 6 is moveable between the closed position shown in Fig. 1a and an open position, whereby in the open position the valve 6 is separated from the valve seat 8 and fluid is therefore able to flow from the inlet holes 14 to the second section 4.

The second section 4 comprises a tube 15, leading from the valve seat 8 to a fluid outlet 16. It will be understood by those skilled in the art that, when the valve 6 is in an open position, fluid is directed through the inlet holes 14, through the at least one orifice disposed in the valve seat 8, through the tube 15 and out of the outlet 16 of the device 1. The flow of fluid through the device 1 will be described in further detail with reference to Fig. 1b.

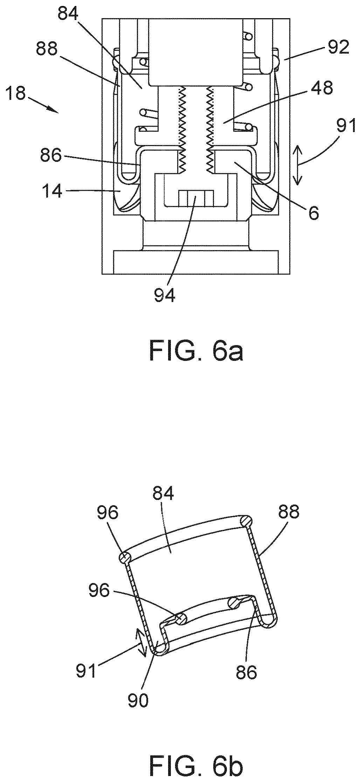

Fig. 1a also shows a seal 18 positioned between the inlet holes 14 and the first section 2 so as to form a sealed compartment within the first section 2, whereby the seal 18 prevents fluid from entering the first section 2 through the inlet holes 14. The seal 18 may comprise any seal capable of preventing fluid flowing from the inlet holes 14 to the first section 2, however in this particular example the seal 18 comprises a silicone membrane 84 which will be described in further detail with reference to Figs. 6a and 6b.

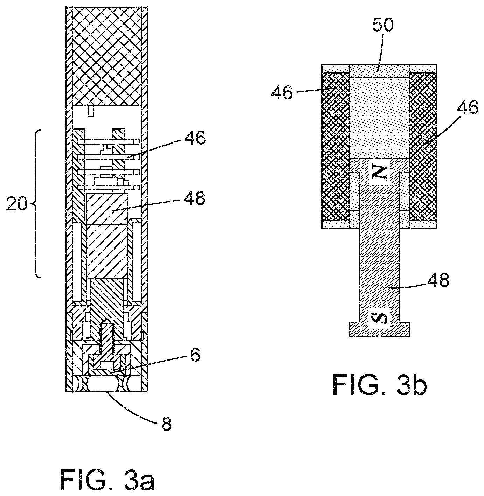

Contained within the sealed compartment within the first section 2 are an actuator 20, a battery 22 and an electronic control circuit 24. The actuator 20 is arranged to impart force to the valve 6 in order move it from an open position to a closed position, or vice versa. Further detail regarding an example actuator 20 will be described in further detail with reference to Figs. 3a and 3b. The battery 22 acts as the power source for the actuator 20 and the electronic control circuit 24. The battery 22 may comprise any type of battery that meets regulatory and/or safety considerations that may be applicable for such a device.

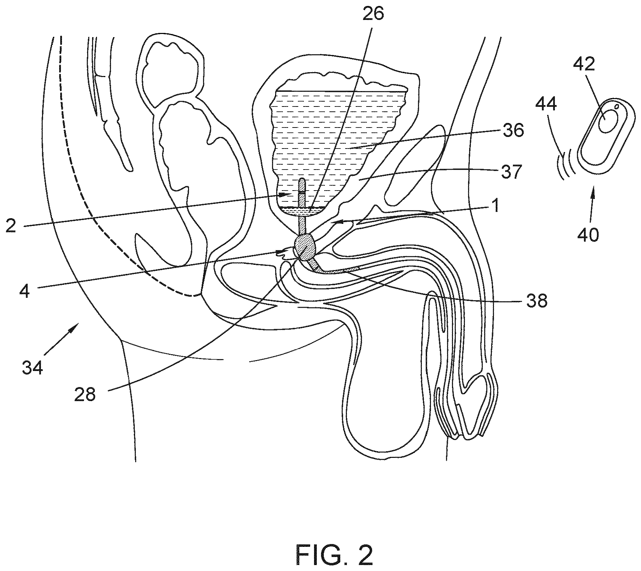

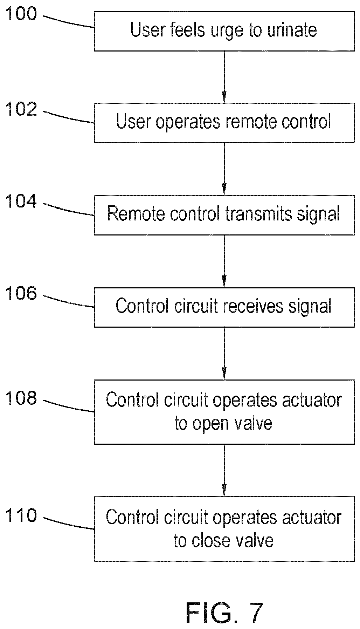

The electronic control circuit 24 is arranged to operate the actuator 20, and thus by extension operate the valve 6. The electronic circuit 24 is further arranged to operate the actuator in response to a wireless signal transmitted by a remote control 40. Thus this control circuit 24 comprises circuitry to receive and decode a radio frequency (RF) signal. In this example, the RF signal is transmitted by a remote control 40, as shown in Fig. 2. The operation of the electronic control circuit 24 will be described in further detail with reference to Fig. 7.

The electronic control circuit 24 may in some embodiments comprise wireless charging circuitry (not shown) for charging the battery 22 while the device 1 is in situ (i.e. installed in a user thereof). In such embodiments, the electronic control circuit 24 may comprise circuitry to receive an RF signal and use the received signal power in order to generate a current for charging the battery 22. This may enable the device 1 to remain installed in a user for longer periods of time than in embodiments where no wireless charging capability is included in the electronic control circuit 24. Furthermore, the inclusion of the wireless charging circuitry in the electronic control circuit 24 may enable the use of a physically smaller battery 22 (due to decreased battery life requirements), thereby enabling a reduction in the physical size of the device. This may help improve user comfort. Users may be provided with a wireless charger and battery pack in order to allow for charging the battery whilst users are travelling or otherwise unable to reach a mains electricity supply.

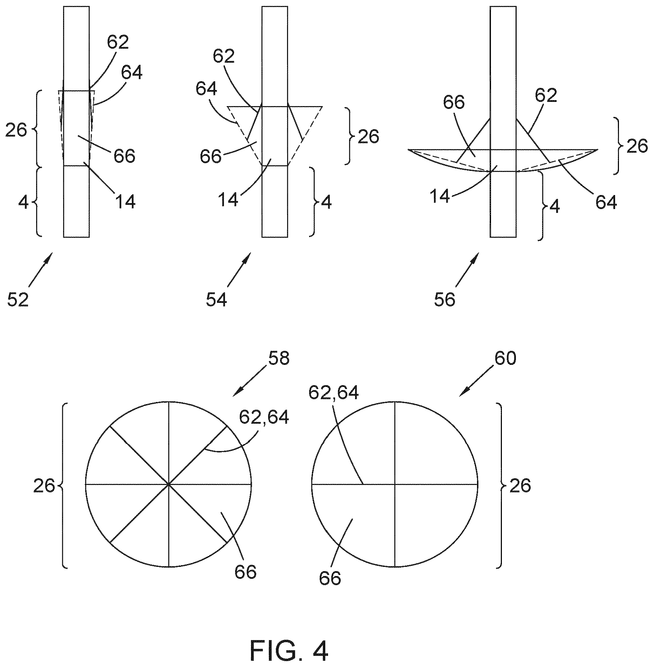

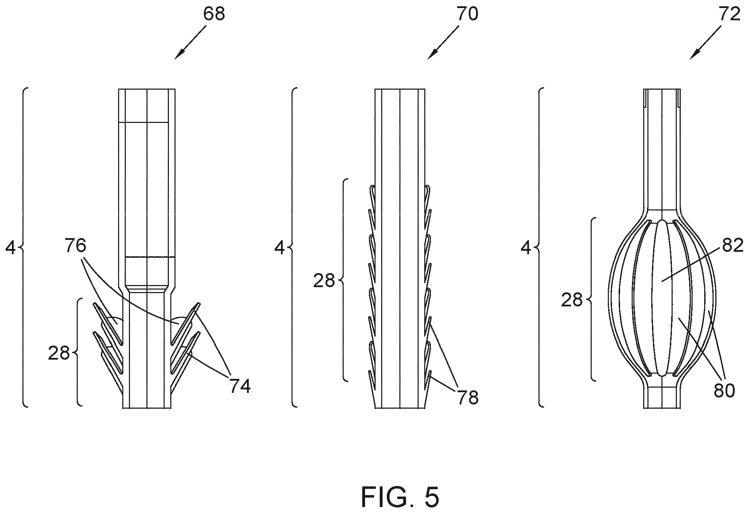

The urinary flow control device 1 further comprises a first positioning structure 26 and a second positioning structure 28 arranged to hold the device 1 in place in the body 34 when in use. The first positioning structure 26 will be described in further detail with reference to Figs. 2 and 4, and the second positioning 28 structure will be described in further detail with reference to Figs. 2 and 5.

Fig. 1b shows an example diagram of fluid flow through the urinary control device 1, when the valve 6 is in an open position. Flow indicators 30 show an example of how fluid surrounding the device 1 may flow into the inlet holes 14. After flowing through inlet holes 14, the fluid flows through the at least one orifice disposed in the valve seat 8 into the tube 15, and out of the fluid outlet 16 - shown by flow indicator 32.

Fig. 2 shows a cross sectional view of a user's body 34 when the device 1 is in use. The device 1 is shown positioned between the bladder 36 and the urethra 38, with the first section 2 positioned in the bladder 36 and the second section 4 positioned in the urethra 38. The device 1 is held in place as a result of the first positioning structure 26 preventing the device from moving further into the urethra 38 and the second positioning structure 28 preventing the device from moving further into the bladder 36. Also shown is a remote control 40, which is manually operated by the user 34. When the user 34 feels the urge to urinate, they can press the button 42 on the remote control 40, which emits an RF signal 44 which is then received and decoded by the electronic control circuit 24, which in turn operates the actuator 20 (and therefore valve 8) in response. The process by which a user 34 may use the device 1 is described further with reference to Fig. 7.

In the example shown in Fig. 2 the first positioning structure 26, when deployed, is arranged to form a bowl or upside down umbrella shape that follows the shape of the bladder wall 37. This arrangement directs fluid towards the inlet holes 14 of the device 1, preventing fluid from leaving the bladder 36 by passing around the outside of the device 1. Additionally, this arrangement means that the inlet holes 14 of the device 1 can be located very close to the interface between the bladder 36 and the urethra 38 (i.e. the bladder neck), and the space between the first positioning structure 26 and bladder wall 37 is minimised. This arrangement allows the bladder 36 to be substantially fully voided, whereby the amount of residual urine left in the bladder 36 is minimised after a user 34 attempts a full voiding of the bladder 36. This helps to prevent, for example, the development of urinary tract infections (UTIs) that may develop as a result of residual urine.

In the example shown in Fig. 2 the second positioning structure 28 comprises a balloon-type structure 28. When inflated, the balloon-type structure 28 holds the device in place as a result of friction between the balloon-type structure 28 and the walls of the urethra 38.

It will be understood that the first and second positioning structures 26 and 28 shown are not limiting, but that various different implementations of the first and second positioning structures 26 and 28 may be used in accordance with the present invention, as will be described in further detail with reference to Figs. 4 and 5. Embodiments of the first and second positioning structures 26 and 28, however, are moveable between two positions: a stored position and a deployed position. In the stored position, the first and second positioning structures are arranged to minimise the maximum footprint of the device 1 (i.e. its diameter) so as to allow the device to be inserted into the body 34 through the urethra 38, using an insertion tool. In the deployed position, the first and second positioning structures 26 and 28 perform the functions described above. The first and second positioning structures 26 and 28 are shown in their respective deployed positions in Fig. 2.

Fig. 3a shows a diagram of an example structure of the actuator 20. In this example, the actuator 20 comprises a solenoid actuator arranged to impart a linear force to the valve 6. The actuator 20 comprises a coil of wire 46 surrounding an empty space wherein a plunger 48 can move linearly in a longitudinal direction. The plunger 48 comprises a permanent magnet with a fixed polarity. It will be understood by those skilled in the art that when an electric current is passed through the coil 46, a magnetic field is generated that interacts with the magnetic field of the permanent magnet plunger 48 in such a way that a linear force, parallel to the longitudinal axis of the device 1, is imparted on the plunger 48. It will also be understood by those skilled in the art that the direction of the force applied to the plunger 48 by the coil 46 is dependent on the direction of current flow through the coil and the polarity of the plunger 48. Thus, the solenoid actuator 20 may be used to move the plunger 48, and therefore the valve 6, in two possible directions - away from the valve seat 8 or towards the valve seat 8, by changing the direction of current flow in the coil 46.

Fig. 3b shows a simplified diagram of the solenoid actuator 20 in order to help demonstrate its functionality. A stopper 50 is shown in Fig. 3b, wherein the stopper 50 is arranged to prevent the plunger 48 from moving further than a predetermined distance in a given direction. The stopper 50 is not necessarily physically shaped and located as shown in Fig. 3b, but instead may be shaped in any way and positioned at any position in the device 1 whereby it may prevent excess motion of the plunger in a given direction.

In a set of embodiments of the present invention, the actuator 20 comprises a latching (bi-stable) solenoid actuator. The operating principles of a latching solenoid are the same as those described above with reference to Figs. 3a and 3b, however a latching solenoid further comprises at least one spring, permanent magnet, latch, or any other source of force which doesn't require electrical power, that forces the plunger 48 to remain in a given position when no current is passed through the coil. The latching solenoid actuator 20 is consequently arranged to have two stable positions - one corresponding to the valve 6 being in an open position (i.e. separated from the valve seat 8) and the other corresponding to the valve 6 being in a closed position (i.e. in contact with the valve seat 8). Hence it will be understood by those skilled in the art that current need only be passed through the coil 46 when it is desired to move the valve from the open position to the closed position, or vice versa. No electrical current is required to maintain the valve in either the open position or the closed position.

Fig. 4 shows schematic diagrams illustrating the design and operation of an example of the first positioning structure 26. In Fig. 4, reference numbers 52, 54 and 56 indicate a side view of the first positioning structure 26 at various stages of deployment, and reference numbers 58 and 60 show top-down views of two possible embodiments of the first positioning structure 26 when in a deployed configuration.

In the examples shown in Fig. 4, the first positioning structure 26 comprises a plurality of arms 64 pivotably mounted at one end to the device 1, wherein each arm 64 further comprises a support strut 62 attached at one end to an intermediate point on the arm 64 and attached in slidable manner at the opposite end to the device 1. Reference number 58 shows a top-down view of an embodiment in which the first positioning structure 26 comprises eight arms 64, each with a respective support strut 62. Reference number 60 shows a top-down view of a second embodiment in which the first positioning structure 26 comprises four arms 64, each with a respective support strut 62. It will be understood by those skilled in the art that the number of arms 64 (and therefore support struts 62) is not limited to that shown in the examples of Fig. 4, but may be any positive integer. In this example, the arms 64 and support struts 62 are made of titanium due to its high corrosion resistance and high biocompatibility. Attached to the arms 64 is a silicone skirt 66.

Reference number 52 shows the first positioning structure 26 in a stored configuration. For the sake of illustration, the arms 64 and support struts 62 are shown to be slightly displaced from the main body of the device 1, however it will be understood by those skilled in the art that in the stored configuration the arms 64 and struts 62 may be flush with the main body of the device 1 to facilitate insertion and removal of the device 1 with minimal irritation of the urethra 38. In the stored configuration, the support structure 26 is arranged to minimise the footprint of the device 1 so as to allow the device 1 to be inserted into the body 34 through the urethra 38.

Reference number 54 shows the first positioning structure 26 in an intermediate configuration, wherein the positioning structure 26 is mid-transition between a stored configuration and a deployed configuration, or vice versa. It can be seen in this state that the ends of support struts 62 that are slidably attached to the device 1 have moved down relative to the device 1, causing the arms 64 to pivot away from the main body of the device 1. Reference number 56 shows the first positioning structure 26 in a deployed configuration, wherein the ends of the support struts 62 that are slidably attached to the device 1 have moved down relative to the device 1 to by a maximum amount (wherein further downward motion is limited by, for example, a latch or stop. As a result, the arms 64 have pivoted away from the device 1 to a maximum angle.

The silicone skirt 66 is attached to the arms 64 in such a way that in the deployed configuration shown by reference numbers 56, 58 and 60, the skirt 66 is deployed into a bowl shape (or an upside down umbrella shape). The arms 64 and skirt 66 in this configuration prevent the device 1 from moving out of the bladder 36. This configuration also allows the skirt 66 to conform to the shape of the lining of the bladder 36, forcing fluid to flow into the inlet holes 14 and preventing fluid from flowing from the bladder 36 to the urethra 38 under the skirt 66 and around the outside of the device 1. In the stored and intermediate configurations shown by reference numbers 52 and 54, the skirt 66 is partly folded (i.e. it is not taut). With this arrangement, no significant extra force is required to move the first position structure 26 from the stored configuration to the deployed configuration, or vice versa (as would be required if the skirt 66 were elastic and needed to be stretched into the deployed configuration). The skirt 66 may be made of other biocompatible materials with similar properties to silicone, e.g. thermoplastic elastomer (TPE).

Fig. 5 shows three examples of the second positioning structure 28 each located on the outside of the second section 4. In the example shown by reference number 68, the second positioning structure 28 comprises a plurality of fins 74 pivotably attached to the device 1, arranged to pivot away from the device 1 when in a deployed configuration (as shown in the figure). When in the deployed configuration, the fins 74 embed slightly into the lining of the urethra 38, preventing the device 1 from moving further into the bladder 36 when in use. The flaps 76 prevent the fins 74 from pivoting beyond a desired angle from the device 1 when in the deployed configuration. The fins 74 are arranged also to adopt a stored configuration (not shown), whereby the fins 74 lie flush to the device 1 in order to minimise the footprint (diameter) of the device 1, allowing the device 1 to be inserted into the body 34 through the urethra 38. It will be understood by those skilled in the art that the number of fins 74 is not limiting, but may be any number. The fins 74 are positioned on either side of the device 1 in the example shown by reference number 68, however it will be understood by those skilled in the art that fins 74 may be positioned at any point on the circumference of the side wall of the device 1. In the example shown by reference number 68, the fins 74 and flaps 76 are made of a soft, biocompatible material, so as not to damage the lining of the urethra 38 when in the deployed configuration. In some examples the fins 75 and flaps 76 may be made from silicone.

In the example shown by reference number 70, the second positioning structure 28 comprises a plurality of smaller fins 78 pivotably attached to the device 1, arranged to pivot away from the device 1 when in a deployed configuration (as shown in the figure). When in the deployed configuration, the fins 78 embed slightly into the lining of the urethra 38, preventing the device 1 from moving further into the bladder 36 when in use. The smaller fins 78 are arranged to also adopt a stored configuration (not shown), whereby the fins 78 lie flush to the device 1 in order to minimise the footprint of the device 1, allowing the device 1 to be inserted into the body 34 through the urethra 38. It will be understood by those skilled in the art that the number of fins 78 is not limiting, but may be any number. The fins 78 are positioned on either side of the device 1 in the example shown by reference number 70, however it will be understood by those skilled in the art that fins 78 may be positioned at any point on the circumference of the side wall of the device 1. In the example shown in 70, the fins 78 are made of a soft, biocompatible material (such as silicone), so as not to damage the lining of the urethra 38 when in the deployed configuration.

In the example shown by reference number 72, the second positioning structure comprises a plurality of expandable segments (or ribs) 80 arranged to expand to form a cavity 82 when in a deployed configuration (as shown in the figure). When in the deployed configuration, the segments 80 push against the lining of the urethra 38, preventing the device 1 from moving further into the bladder 36 when in use. The segments 80 are arranged to also adopt a stored configuration (not shown), whereby the segments 80 lie flush to the device 1 in order to minimise the footprint of the device 1, allowing the device 1 to be inserted into the body 34 through the urethra 38. The gaps that form between the segments 80 when in the deployed configuration, may act as an additional fluid outlet 16 for the device 1. In the example shown by reference number 72, the segments 80 are made of a soft, biocompatible material (e.g. silicone), so as not to damage the lining of the urethra 38 when in the deployed configuration. In this example, the segments 80 may be expanded and contracted (deployed or stored) by lengthening or shortening the second section 4.

Figs. 6a and 6b show an embodiment of the seal 18, whereby the seal 18 comprises a silicone membrane 84. It will be understood by those skilled in the art that the seal 18 is not limited to the silicone membrane shown in Figs. 6a and 6b, but may comprise any type of seal capable of sealing the first section 2 from the fluid flowing into the inlet 14 of the device 1. It will also be understood by those skilled in the art that the membrane 84 is not limited to silicone, but may be made out of any appropriate, impermeable material, e.g. polyurethane.

As shown in Fig. 6b, the membrane 84 is formed as a tube, with one end of the tube folded back in on itself, creating an inner tube circumference 86 and an outer tube circumference 88. In doing this, part of the membrane 84 forms a cut-away of a toroid 90, with an overlap 91 between the inner tube circumference 86 and the outer tube circumference 88.

As shown in Fig. 6a, the outer tube circumference 88 of the membrane 84 is secured to the inner wall 92 of the device 1 in a manner so as to prevent fluid from passing between the outer tube circumference 88 of the membrane 84 and the inner wall 92 of the device 1 into the first section 2. The inner tube circumference 86 of the membrane 84 is secured between the valve 6 and plunger 48 using a screw 94 in a manner so as to prevent fluid from passing between the inner tube circumference 86 of the membrane 84 and the valve 6 into the first section 2. It will be understood by those skilled in the art that securing the membrane 84 to the device 1 in this way allows a seal 18 to be formed between the fluid inlet holes 14 and the first section 2. In this embodiment the membrane 84 has integral O-rings 96 formed at each end, these integral O-rings being sandwiched and compressed by appropriate structures on the inner wall 92 or between the valve 6 and plunger 48, thereby forming an effective fluid-tight seal at each open end of the membrane tube. Although not shown in Fig. 6b, it may be appreciated that the membrane 84 may be formed as a conical tube that tapers from a large end to a small end so that the small end can be more easily folded back inside the large end.

This arrangement for the seal 18 provides the ability to seal off the first section 2 from the inlet holes 14 in such a way that the valve 6 and piston 48 are able to move substantially uninhibited by the seal 18. When the valve 6 is moved from an open position to a closed position, or vice versa, the membrane 84 deforms in a rolling motion, wherein the movement of the valve changes the length of the overlap 91. This rolling motion introduces very little additional friction or stretch to the motion of the valve, and thus helps reduce the energy required by the actuator 20 to move the valve 6. Reducing the energy required for this movement increases the operational lifespan of the device 1 which is largely determined by the battery life. The rolling motion is also non-damaging to the membrane 84 such that it can be deformed back and forth a very large number of times with low risk of failure.

In this arrangement the body of the first section 2 (the side walls 92 and the closed distal end of the device 1) together with the seal 18 (membrane 84) form a sealed compartment which is sealed in a fluid-tight manner such that the actuator and the battery and electronic control circuit that are used to power and control the actuator are all protected from contact with urine during use.

It will be understood by those skilled in the art that the seal 18 is not limited to the membrane 84 as shown in Figs. 6a and 6b, but may comprise any appropriate sealing mechanism that prevents fluid from flowing from the inlet holes 14 to the first portion 2 of the device 1.

Fig. 7 shows a flowchart of an example process by which the device 1 is operated by a user 34, when the device 1 is in use. Also contained within the flowchart are operations performed by the electronic control circuit 24. At step 100, the user 34 feels the urge to urinate. In order to do so, the user 34 operates the remote control 40 (step 102), which in turn transmits a radio frequency (RF) signal 44 (step 104). The signal 44 is then received by the electronic control circuit 24 (step 106), which may comprise a number of standard modules such as processors, oscillators, filters, amplifiers, digital to analogue converters and analogue to digital converters, as may be required for the particular signal and protocols.

The control circuit 24, in response to the signal 44, operates the actuator 20 in order to open the valve 6 (step 108), allowing urine to flow from the bladder 36, through the device 1, through the urethra 38 and out of the body 34. In this example, the control circuit 24 comprises an electrolytic capacitor and a voltage booster circuit portion. The control circuit 24 charges the capacitor using the voltage booster circuit portion. In this way, the capacitor can be charged to a higher voltage than the battery and so can store a large amount of energy sufficient to generate a pulse to operate the solenoid. The capacitor is then discharged in order to provide a large current to the solenoid actuator 24 in order to move the valve 6. It will be understood by those skilled in the art that the control circuit 24 is not limited to this example, but may comprise any electronic circuit capable of providing current to the actuator 20. In this example, the control circuit further comprises means for monitoring the remaining energy in the battery 22.

Either when the user 34 operates the remote control 40 or when the device 1 senses that the bladder has been substantially fully voided, or a set time after initial triggering, the control circuit 24 operates the actuator 20 to return the valve 6 to the closed position (step 106), preventing further urine flow.

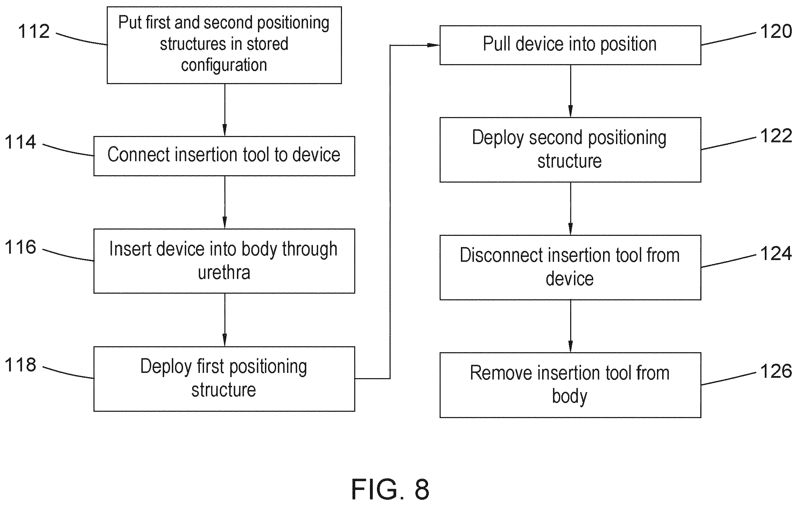

Fig. 8 shows a flowchart of an example process by which the device 1 is inserted into the desired position (as shown in Fig. 2) in a user's body 34. Initially, at step 112, a trained person (e.g. a doctor) will put the first positioning structure 26 and the second positioning structure 28 into their respective stored configurations. Step 112 may not be needed if the device 1 is provided ready for insertion with the first positioning structure 26 and the second positioning structure 28 already in their respective stored configurations. Next, at step 114, the trained person connects an insertion tool to the device 1. This may be accomplished through the use of a physical latch, magnetic latch, or any other means of temporary attachment. At step 116, the trained person inserts the device 1 into a desired position within the user 34 through the urethra 38, slightly further into the bladder 36 than the desired operating position.

At step 118, the trained person uses the insertion tool (or a dedicated deployment tool) to deploy the first positioning structure 26. This may be accomplished in numerous ways, for example through a mechanical connection between the insertion tool and the first positioning structure 26 or using a signal being transmitted to the electronic control circuit 24, which in turn operates the first positioning structure 26. At step 120, the trained person pulls the device 1 back out of the bladder 36 slightly until the device 1 is in the desired location.

Next, at step 122, the trained person deploys the second positioning structure 28. Again, this may be accomplished in numerous ways, for example through a mechanical connection between the insertion tool and the second positioning structure 28 or using a signal being transmitted to the electronic control circuit 24, which in turn operates the second positioning structure 28. At step 124, the trained person disconnects the insertion tool from the device 1. Finally, at step 126, the trained person removes the insertion tool from the body 34 through the urethra 38, leaving the device 1 anchored in the desired position in the body 34.

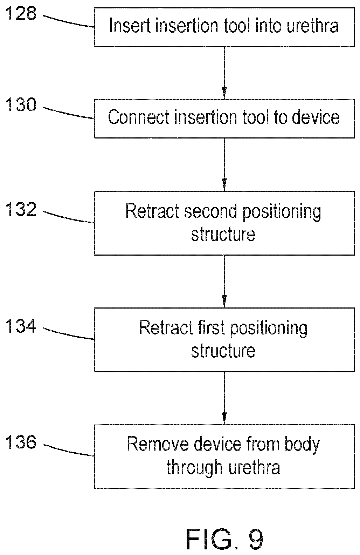

Fig. 9 shows a flowchart of an example process by which the device 1 is removed from a user's body 34. Initially, at step 128, a trained person (e.g. a doctor) inserts the insertion tool (or a removal tool) into the body 34 through the urethra 38. The trained person then, at step 130, connects the insertion tool to the device 1. This may be accomplished through the use of a physical latch, magnetic latch, or any other means of temporary attachment. At step 132, the trained person retracts the second positioning structure 28 back to the stored configuration. At step 134, the trained person retracts the first positioning structure 26 back to the stored configuration. Both retractions can be carried out with the help of the insertion tool (or removal tool) or with one or more dedicated tools that retract the positioning structure(s) 26, 28 back to the stored configuration. Finally, at step 136, the trained person removes the device 1 from the user's body 34 through the urethra 38.

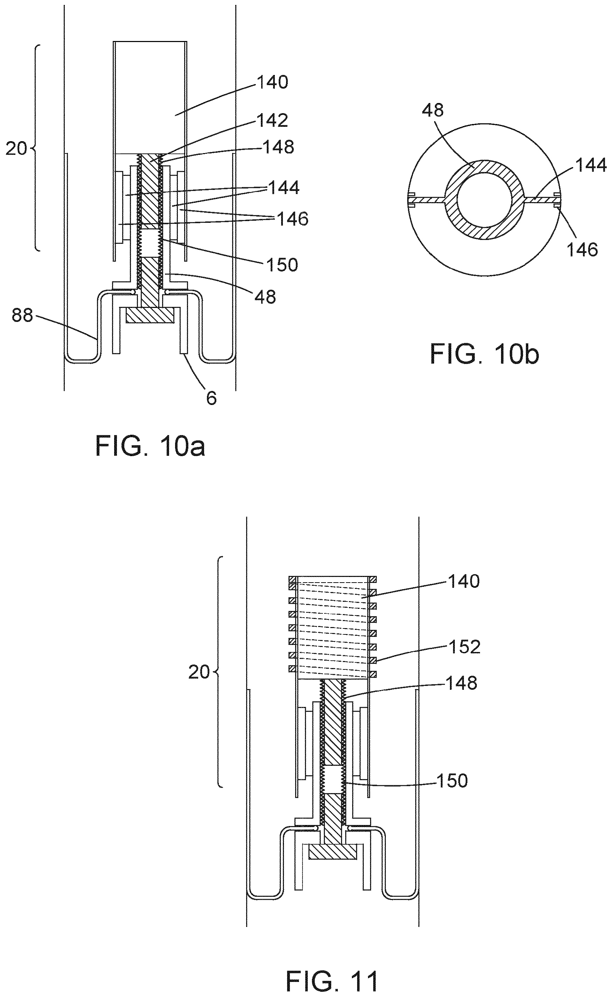

Fig. 10a shows a side-on, cross-sectional diagram of a second example structure of the actuator 20. In this example, the actuator 20 comprises a screw-based actuator, driven by an electric rotational motor 140, arranged to impart a linear force to the valve 6. The actuator 20 comprises an electric motor 140, a screw 142, a plunger 48, two guiding members 144 and two guide rails 146. The plunger 48 in this example is substantially tube-like in shape - i.e. the plunger 48 is substantially cylindrical in shape and features a bore extending from the distal end thereof to the proximal end thereof. A first screw thread 148 is cut into the external curved surface of the screw 142, and a second screw thread 150 is cut into the internal curved surface of the bore of the plunger 48. The second screw thread 150 of the plunger 48 is arranged to moveably engage with the first screw thread 148 of the screw 142. The first and second screw threads 148 & 150 may be arranged to slide relative to one another with low friction.

The plunger 48 features two guiding members 144 positioned on opposite sides of the external curved surface of the plunger 48. The two guiding members 144 are each arranged to moveably engage with one of the two guide rails 146 which are positioned on either side of the plunger 48. The guide rails 146 allow the guiding members 144 to slide along an axis substantially parallel to the longitudinal axis of the device 1 with low resistance, but prevent lateral twisting motion thereof (i.e. into/out of the page relative to Fig. 10a). This is illustrated more clearly in Fig. 10b which shows a top-down, cross-sectional diagram of the second example actuator 20 structure shown in Fig. 10a and illustrates how the guiding members 144 and the guide rails 146 interlock. It will be appreciated that the actuator 20 shown in this example is not limited to two guiding members 144 and guide rails 146, but may comprise any suitable number of guiding members 144 and a corresponding number of guide rails 146. For example a single guiding member 144 and guide rail 146 may suffice, or three or more guiding members 144 and guide rails 146 may be provided.

When activated, the electric motor 140 imparts a rotational driving force to the screw 142. The electric motor 140 is capable of rotating the screw 142 in a clockwise or an anti-clockwise direction, dependent on a control signal provided thereto by the electronic control circuit 24 (not shown in Fig. 10a for simplicity). As the screw 142 rotates and the thread 148 thereof engages with the thread 150 of the plunger 48, the plunger 48 is caused to move along an axis substantially parallel to its length. This is because the guiding members 144 and guide rails 146 prevent rotation of the plunger 48, thereby causing the screw threads 148 & 150 of the screw 142 and the plunger 48 to slide over each other. This imparts a linear motion to the plunger 48. As a result, the rotational electric motor 140 imparts a driving force to the valve 6, allowing it to be moved towards or away from the valve seat 8 (not shown in Fig. 10a for simplicity) as required for operation of the device 1.

The screw-based actuator 20 shown in Fig. 10a advantageously allows the rate at which the valve 6 moves to be controlled more precisely than the solenoid actuator 20 shown in Fig. 3a. This may improve the comfort of users of the device 1 by reducing vibration and/or noise. Furthermore, the screw-based actuator 20 may draw less power on each actuation (i.e. an opening or closing of the valve 6) than the solenoid actuator 20 shown in Fig. 3a. This may have the benefit of improving the battery life of the device 1. The electric motor 140 may be provided with a gearbox in order to reduce the motor speed and increase the torque to an optimal level for sealing of the valve and for overcoming any resistive forces such as may be provided by a seal or membrane that prevents fluid contacting the actuator 20. This may help improve a user's comfort level when using the device 1.

Fig. 11 shows a diagram of the example actuator 20 shown in Figs. 10a and 10b, further comprising a coil capacitor 152. In this example, the coil capacitor 152 is helically wrapped around the electric motor 140. It will be appreciated that the actuator 20 may equally comprise a cylindrical capacitor, or any other appropriately shaped capacitor, in place of the coil capacitor 152. A cylindrical capacitor may be wrapped around the electric motor 140. Any type of capacitor may be positioned adjacent to one or more other components of the actuator 20 along the longitudinal axis of the device 1. The capacitor 152 may act as an intermediate charge storage device between the battery 22 and the electric motor 140 - the capacitor 152 may supply power to the motor 140 when discharging and may draw power from the battery 22 in order to charge. Alternatively, the coil capacitor 152 may be used to charge the battery 22, and the battery 22 may supply power to the motor 140. The inclusion of the capacitor 152 may improve the battery life of the device 1, as it may be pre-charged before the device 1 is inserted into a user, thereby increasing the initial storage capacity of the device 1. The battery life of the device 1 may be improved by 20-30% by including the capacitor 152 in addition to the battery 22. Conveniently, by including the capacitor 152 and thereby extending the battery life of the device 1, the size of the battery 22 may be reduced accordingly. This may enable a reduction in the physical size of the device 1, which may improve user comfort levels. In particular, this may help reduce the length of the urinary control device along its longitudinal axis by utilising unoccupied space in the radial direction, or by slightly increasing a radius of the device as a trade-off for the decrease in length. It is particularly desirable to reduce the length of the urinary control device along its longitudinal axis, as bladders may collapse (e.g. when empty) onto the device when installed in a user if the device is too long, thereby causing discomfort or even damage to the bladder tissue.

Alternatively, the capacitor 152 may be provided in place of the battery 22 and therefore act as the sole power supply for the device 1. This may have the benefit of reducing the size of the device 1 (and therefore improving user comfort), at the cost of decreasing battery life.

It will be understood that the capacitor 152 may be similarly included in a device 1 comprising a solenoid actuator as shown in Figs. 3a and 3b. It will also be understood that the capacitor 152 is not limited to a coil capacitor as shown in this example, but may comprise any suitable type of capacitor, provided it is small enough to fit into the device 1. While wrapping the capacitor 152 around the motor 140 provides a convenient location for it while not requiring a substantial increase in the physical size of the device 1, the location of the capacitor 152 is not limited as such. For example, the capacitor 152 may equally be wrapped around the battery 22 (not shown) or may be placed alongside other components. The size and shape of capacitors may be varied to suit the available space in the device 1.

It will be appreciated that the above embodiments are described in order to illustrate some ways of putting the invention into effect. However, many variations of the above embodiments may be made without departing from the scope of the invention which is defined by the appended claims.

## Claims

1. A device (1) for controlling urinary flow, comprising:
a fluid inlet (14);
a fluid outlet (16);
a valve (6) movable between an open position in which fluid can flow from the fluid inlet (14) to the fluid outlet (16) and a closed position in which fluid flow is blocked between the fluid inlet (14) and the fluid outlet (16);
a valve seat (8) located between the fluid inlet (14) and the fluid outlet (16) and arranged such that the valve (6) engages with the valve seat (8) in the closed position; and
an actuator (20) operable to move the valve (6) between the open position and the closed position;
**characterised in that**:
the actuator (20) is positioned on a first side of the fluid inlet (14);
the fluid outlet is positioned on a second, opposite side of the fluid inlet (14); and
the valve seat (8) is located close to the fluid inlet (14) such that the valve (6) is operable to move substantially out of the flow path (30, 32) when the valve (6) is in the open position.

2. A device (1) as claimed in claim 1, comprising an outlet tube (15) extending between the fluid inlet (14) and the fluid outlet (16).

3. A device (1) as claimed in claim 1 or 2, wherein the device (1) is elongate and comprises a first section (2) comprising the actuator (20) and a second section (4) comprising the fluid outlet (16); wherein the fluid inlet (14) is formed in a side wall of the device (1) at a position between the first section (2) and the second section (4); and
wherein the device (1) is insertable into a urethra (38) and is arranged such that, in use, the first section (2) is located in a bladder (36) and the second section (4) is located in the urethra (38).

4. A device (1) as claimed in any preceding claim, further comprising a seal (18) arranged between the fluid inlet (14) and the actuator (20) to prevent fluid flowing from the fluid inlet (14) to the actuator (20);
preferably wherein the actuator (20) is located in a sealed compartment, the sealed compartment at least partially formed by the seal (18).

5. A device (1) as claimed in claim 4, wherein the seal (18) comprises a membrane (84) extending between the valve (6) and an inner wall (92) of the device (1), the membrane (84) arranged to deform as the valve (6) moves between the open position and the closed position.

6. A device (1) as claimed in claim 5, wherein the membrane (84) is arranged in a folded configuration such that the membrane (84) is partly folded back on itself so as to create an overlap (91) and such that movement of the valve (6) between the open position and the closed position changes the amount of overlap (91); preferably wherein the membrane (84) is at least partly in the form of a tube with one end folded back inside itself.

7. A device (1) as claimed in any preceding claim, wherein the device (1) further comprises a first positioning structure (26) movable between a stored configuration (52) and a deployed configuration (56) and wherein, in use, the first positioning structure (26) prevents the device (1) from exiting the bladder (36) through the urethra (38);
preferably wherein the first positioning structure (26) is designed to contact a bladder wall (37) when in the deployed configuration (56).

8. A device (1) as claimed in claim 7, wherein the first positioning structure (26) comprises a skirt (66).

9. A device (1) as claimed in claim 7 or 8, wherein the first positioning structure (26) is attached to the device (1) at a position between the fluid inlet (14) and the fluid outlet (16) and adjacent to the fluid inlet (14).

10. A device (1) as claimed in any of claims 7 to 9, wherein the first positioning structure (26) is arranged when in the deployed configuration (56) to position the fluid inlet (14) adjacent to a urethral opening in the bladder (36).

11. A device (1) as claimed in any preceding claim, wherein the device (1) further comprises a second positioning structure (28) movable between a stored configuration and a deployed configuration and wherein, in use, the second positioning structure (28) is arranged to prevent movement of the device (1) towards the bladder (36);
preferably wherein the second positioning structure (28) is designed to contact a urethra wall when in the deployed configuration.

12. A device (1) as claimed in any preceding claim, wherein the actuator (20) comprises a solenoid (46, 48) arranged to impart a linear force on the valve (6) to move it between the open position and the closed position;
preferably wherein the solenoid (46, 48) is a latching solenoid (46, 48), which is able to maintain two or more set positions without constant application of electrical power.

13. A device (1) as claimed in any preceding claim, wherein the actuator (20) comprises a battery (22) and an electronic control circuit (24).

14. A device (1) as claimed in claim 13, wherein the electronic control circuit (24) is arranged to receive a wireless signal (44) from an external source (40) and operate the valve (6) in response to said signal (44);
preferably wherein the electronic control circuit (24) is arranged to receive a radio frequency (RF) signal (44) from a remote control (40) that is manually operable by a user (34) of the device (1).

15. A device (1) as claimed in any preceding claim, wherein the actuator (20) comprises a capacitor (152).

## Patentansprüche

1. Vorrichtung (1) zum Steuern eines Harnflusses, umfassend:
einen Fluideinlass (14);
einen Fluidauslass (16);
ein Ventil (6), das zwischen einer offenen Position, in der Fluid von dem Fluideinlass (14) zu dem Fluidauslass (16) fließen kann, und einer geschlossenen Position, in der der Fluidfluss zwischen dem Fluideinlass (14) und dem Fluidauslass (16) blockiert ist, bewegbar ist;
einen Ventilsitz (8), der zwischen dem Fluideinlass (14) und dem Fluidauslass (16) lokalisiert und so angeordnet ist, dass das Ventil (6) in der geschlossenen Position mit dem Ventilsitz (8) in Eingriff kommt; und
einen Aktuator (20), der betrieben werden kann, um das Ventil (6) zwischen der offenen Position und der geschlossenen Position zu bewegen;
**dadurch gekennzeichnet, dass**:
der Aktuator (20) auf einer ersten Seite des Fluideinlasses (14) positioniert ist;
der Fluidauslass auf einer zweiten, gegenüberliegenden Seite des Fluideinlasses (14) positioniert ist; und
der Ventilsitz (8) in der Nähe des Fluideinlasses (14) lokalisiert ist, so dass das Ventil (6) betrieben werden kann, um sich im Wesentlichen aus dem Flusspfad (30, 32) zu bewegen, wenn das Ventil (6) in der offenen Position ist.

2. Vorrichtung (1) nach Anspruch 1, umfassend einen Auslassschlauch (15), der sich zwischen dem Fluideinlass (14) und dem Fluidauslass (16) erstreckt.

3. Vorrichtung (1) nach Anspruch 1 oder 2, wobei die Vorrichtung (1) länglich ist und eine erste Sektion (2), die den Aktuator (20) umfasst, und eine zweite Sektion (4), die den Fluidauslass (16) umfasst, umfasst, wobei der Fluideinlass (14) in einer Seitenwand der Vorrichtung (1) an einer Position zwischen der ersten Sektion (2) und der zweiten Sektion (4) ausgebildet ist; und
wobei die Vorrichtung (1) in eine Harnröhre (38) eingeführt werden kann und so angeordnet ist, dass bei Verwendung die erste Sektion (2) in einer Harnblase (36) lokalisiert ist und die zweite Sektion (4) in der Harnröhre (38) lokalisiert ist.

4. Vorrichtung (1) nach einem vorstehenden Anspruch, weiter umfassend eine Dichtung (18), die zwischen dem Fluideinlass (14) und dem Aktuator (20) angeordnet ist, um zu verhindern, dass Fluid von dem Fluideinlass (14) zu dem Aktuator (20) fließt;
wobei vorzugsweise der Aktuator (20) in einem abgedichteten Fach lokalisiert ist, wobei das abgedichtete Fach mindestens teilweise durch die Dichtung (18) ausgebildet ist.

5. Vorrichtung (1) nach Anspruch 4, wobei die Dichtung (18) eine Membran (84) umfasst, die sich zwischen dem Ventil (6) und einer Innenwand (92) der Vorrichtung (1) erstreckt, wobei die Membran (84) angeordnet ist, um sich zu verformen, während sich das Ventil (6) zwischen der offenen Position und der geschlossenen Position bewegt.

6. Vorrichtung (1) nach Anspruch 5, wobei die Membran (84) in einer gefalteten Konfiguration angeordnet ist, so dass die Membran (84) teilweise auf sich selbst zurückgefaltet ist, um eine Überlappung (91) zu kreieren, und so, dass Bewegung des Ventils (6) zwischen der offenen Position und der geschlossenen Position das Ausmaß der Überlappung (91) ändert; wobei die Membran (84) vorzugsweise mindestens teilweise in Form eines Schlauchs ausgebildet ist, dessen eines Ende in sich selbst zurückgefaltet ist.

7. Vorrichtung (1) nach einem vorstehenden Anspruch, wobei die Vorrichtung (1) weiter eine erste positionierende Struktur (26) umfasst, die zwischen einer gelagerten Konfiguration (52) und einer entfalteten Konfiguration (56) bewegbar ist, und wobei die erste positionierende Struktur (26) bei Verwendung verhindert, dass die Vorrichtung (1) die Harnblase (36) durch die Harnröhre (38) verlässt;
wobei die erste positionierende Struktur (26) vorzugsweise ausgelegt ist, um eine Harnblasenwand (37) zu kontaktieren, wenn sie sich in der entfalteten Konfiguration (56) befindet.

8. Vorrichtung (1) nach Anspruch 7, wobei die erste positionierende Struktur (26) eine Schürze (66) umfasst.

9. Vorrichtung (1) nach Anspruch 7 oder 8, wobei die erste positionierende Struktur (26) an der Vorrichtung (1) an einer Position zwischen dem Fluideinlass (14) und dem Fluidauslass (16) und angrenzend an den Fluideinlass (14) angebracht ist.

10. Vorrichtung (1) nach einem der Ansprüche 7 bis 9, wobei die erste positionierende Struktur (26) angeordnet ist, wenn sie sich in der entfalteten Konfiguration (56) befindet, um den Fluideinlass (14) angrenzend an eine Harnröhrenöffnung in der Harnblase (36) zu positionieren.

11. Vorrichtung (1) nach einem vorstehenden Anspruch, wobei die Vorrichtung (1) weiter eine zweite positionierende Struktur (28) umfasst, die zwischen einer gelagerten Konfiguration und einer entfalteten Konfiguration beweglich ist, wobei die zweite positionierende Struktur (28) bei Verwendung angeordnet ist, um eine Bewegung der Vorrichtung (1) in Richtung der Harnblase (36) zu verhindern;
wobei vorzugsweise die zweite positionierende Struktur (28) ausgelegt ist, um eine Harnröhrenwand zu kontaktieren, wenn sie sich in der entfalteten Konfiguration befindet.

12. Vorrichtung (1) nach einem vorstehenden Anspruch, wobei der Aktuator (20) ein Solenoid (46, 48) umfasst, das angeordnet ist, um eine lineare Kraft auf das Ventil (6) auszuüben, um es zwischen der offenen Position und der geschlossenen Position zu bewegen;
wobei vorzugsweise das Solenoid (46, 48) ein rastendes Solenoid (46, 48) ist, das in der Lage ist, zwei oder mehr eingestellte Positionen ohne ständiges Anlegen von elektrischer Leistung zu unterhalten.

13. Vorrichtung (1) nach einem vorstehenden Anspruch, wobei der Aktuator (20) eine Batterie (22) und einen elektronischen Steuerschaltkreis (24) umfasst.

14. Vorrichtung (1) nach Anspruch 13, wobei der elektronische Steuerschaltkreis (24) angeordnet ist, um ein drahtloses Signal (44) von einer externen Quelle (40) zu empfangen und das Ventil (6) als Antwort auf dieses Signal (44) zu betreiben;
wobei vorzugsweise der elektronische Steuerschaltkreis (24) angeordnet ist, um ein Hochfrequenz- (RF) -Signal (44) von einer Fernsteuerung (40) zu empfangen, die von einem Benutzer (34) der Vorrichtung (1) manuell betrieben werden kann.

15. Vorrichtung (1) nach einem vorstehenden Anspruch, wobei der Aktuator (20) einen Kondensator (152) umfasst.

## Revendications

1. Dispositif (1) de régulation de débit urinaire, comprenant :
une entrée de fluide (14) ;
une sortie de fluide (16) ;
une valve (6) mobile entre une position ouverte dans laquelle le fluide peut s'écouler de l'entrée de fluide (14) vers la sortie de fluide (16) et une position fermée dans laquelle l'écoulement de fluide est bloqué entre l'entrée de fluide (14) et la sortie de fluide (16) ;
un siège de valve (8) situé entre l'entrée de fluide (14) et la sortie de fluide (16) et agencé de telle sorte que la valve (6) s'engage avec le siège de valve (8) dans la position fermée ; et
un actionneur (20) pouvant être actionné pour déplacer la valve (6) entre la position ouverte et la position fermée ;
**caractérisé en ce que** :
l'actionneur (20) est positionné sur un premier côté de l'entrée de fluide (14) ;
la sortie de fluide est positionnée sur un deuxième côté, opposé, de l'entrée de fluide (14) ; et
le siège de valve (8) est situé à proximité de l'entrée de fluide (14) de telle sorte que la valve (6) peut être actionnée pour se déplacer sensiblement hors du chemin d'écoulement (30, 32) lorsque la valve (6) est en position ouverte.

2. Dispositif (1) selon la revendication 1, comprenant un tube de sortie (15) s'étendant entre l'entrée de fluide (14) et la sortie de fluide (16).

3. Dispositif (1) selon la revendication 1 ou la revendication 2, dans lequel le dispositif (1) est allongé et comprend une première section (2) comprenant l'actionneur (20) et une deuxième section (4) comprenant la sortie de fluide (16) ; dans lequel l'entrée de fluide (14) est formée dans une paroi latérale du dispositif (1) à une position entre la première section (2) et la deuxième section (4) ; et
dans lequel le dispositif (1) peut être inséré dans un urètre (38) et est agencé de telle sorte que, lors de l'utilisation, la première section (2) est située dans la vessie (36) et la deuxième section (4) est située dans l'urètre (38).

4. Dispositif (1) selon l'une quelconque des revendications précédentes, comprenant en outre un joint d'étanchéité (18) disposé entre l'entrée de fluide (14) et l'actionneur (20) pour empêcher le fluide de s'écouler de l'entrée de fluide (14) vers l'actionneur (20) ;
de préférence dans lequel l'actionneur (20) est situé dans un compartiment étanche, le compartiment étanche étant au moins partiellement formé par le joint d'étanchéité (18).

5. Dispositif (1) selon la revendication 4, dans lequel le joint d'étanchéité (18) comprend une membrane (84) s'étendant entre la valve (6) et une paroi intérieure (92) du dispositif (1), la membrane (84) étant agencée pour se déformer lorsque la valve (6) se déplace entre la position ouverte et la position fermée.

6. Dispositif (1) selon la revendication 5, dans lequel la membrane (84) est agencée dans une configuration pliée de telle sorte que la membrane (84) est partiellement repliée sur elle-même afin de créer un chevauchement (91) et de telle sorte que le mouvement de la valve (6) entre la position ouverte et la position fermée modifie la quantité de chevauchement (91) ; de préférence, dans lequel la membrane (84) se présente au moins en partie sous la forme d'un tube dont une extrémité est repliée sur elle-même.

7. Dispositif (1) selon l'une quelconque des revendications précédentes, dans lequel le dispositif (1) comprend en outre une première structure de positionnement (26) mobile entre une configuration stockée (52) et une configuration déployée (56) et dans lequel, en utilisation, la première structure de positionnement (26) empêche le dispositif (1) de sortir de la vessie (36) par l'urètre (38) ;
de préférence dans lequel la première structure de positionnement (26) est conçue pour entrer en contact avec une paroi de la vessie (37) lorsqu'elle est dans la configuration déployée (56).

8. Dispositif (1) selon la revendication 7, dans lequel la première structure de positionnement (26) comprend une jupe (66).

9. Dispositif (1) selon la revendication 7 ou la revendication 8, dans lequel la première structure de positionnement (26) est fixée au dispositif (1) à une position située entre l'entrée de fluide (14) et la sortie de fluide (16) et adjacente à l'entrée de fluide (14).

10. Dispositif (1) selon l'une quelconque des revendications 7 à 9, dans lequel la première structure de positionnement (26) est agencée, lorsqu'elle est dans la configuration déployée (56), pour positionner l'entrée de fluide (14) à proximité d'une ouverture urétrale dans la vessie (36).

11. Dispositif (1) selon l'une quelconque des revendications précédentes, dans lequel le dispositif (1) comprend en outre une deuxième structure de positionnement (28) mobile entre une configuration rangée et une configuration déployée et dans lequel, en utilisation, la deuxième structure de positionnement (28) est agencée pour empêcher le mouvement du dispositif (1) vers la vessie (36) ;
de préférence dans lequel la deuxième structure de positionnement (28) est conçue pour entrer en contact avec une paroi de l'urètre lorsqu'elle est dans la configuration déployée.

12. Dispositif (1) selon l'une quelconque des revendications précédentes, dans lequel l'actionneur (20) comprend un solénoïde (46, 48) agencé pour exercer une force linéaire sur la valve (6) afin de la déplacer entre la position ouverte et la position fermée ;
de préférence dans lequel le solénoïde (46, 48) est un solénoïde à verrouillage (46, 48), qui est capable de maintenir deux ou plusieurs positions définies sans application constante d'énergie électrique.

13. Dispositif (1) selon l'une quelconque des revendications précédentes, dans lequel l'actionneur (20) comprend une batterie (22) et un circuit de commande électronique (24).

14. Dispositif (1) selon la revendication 13, dans lequel le circuit de commande électronique (24) est agencé pour recevoir un signal sans fil (44) provenant d'une source externe (40) et actionner la valve (6) en réponse audit signal (44) ;
de préférence, dans lequel le circuit de commande électronique (24) est agencé pour recevoir un signal radiofréquence (RF) (44) provenant d'une télécommande (40) qui peut être actionnée manuellement par un utilisateur (34) du dispositif (1).

15. Dispositif (1) selon l'une quelconque des revendications précédentes, dans lequel l'actionneur (20) comprend un condensateur (152).
